# EUROPEAN PATENT APPLICATION

(11) **EP 1 820 477 A2**
(43) Date of publication of application: **22.08.2007**
(21) Application number: 07001511.0
(22) Date of filing: 24.01.2007
(51) Int. Cl.: A61F 2/46

(54) **Bioprosthesis preparation and implantation kit and bioprosthesis implantation device**

(30) Priority: 17.02.2006 JP 2006040920; 17.02.2006 JP 2006040921
(71) Applicant: Olympus Biomaterial Corp., Shinjuku-ku Tokyo 163-0914 (JP)
(72) Inventor: Kuroda, Kouichi, Hachioji-shi Tokyo 192-0032 (JP); Inoue, Hiraku, Higashimurayama-shi Tokyo 189-0025 (JP)
(74) Representative: von Hellfeld, Axel

(57) **Abstract**

An object is to implant a bioprosthesis constituted by infiltrating a scaffold material held inside a container with body fluid, such as bone marrow, into an affected side without separating the body fluid, to reduce the burden placed on a patient, and to improve the ease-of-use. A bioprosthesis preparation and implantation kit is provided, the kit including a porous scaffold material; a cylindrical container configured to accommodate the scaffold material, the container having openings at first and second ends; a cap removably attached to the first end of the container so as to seal the opening at the first end; and a blocking member removably attached to the second end of the container so as to seal the opening at the second end, wherein a through-hole for injecting liquid, such as bone marrow, is formed in the cap, the blocking member is inserted into the container and is provided with an insertion section for limiting the accommodation space for the scaffold material to a part near the cap, and at least one air-release hole penetrating in the radial direction is formed in the container close to the tip of the insertion section of the blocking member attached to the container.

## Description

### BACKGROUND OF THE INVENTION

### 1. FIELD OF THE INVENTION

The present invention relates to a bioprosthesis preparation and implantation kit and a bioprosthesis implantation device for preparing a bioprosthesis by infiltrating a porous scaffold with body fluid, such as bone marrow, and implanting the prepared bioprosthesis into an affected site.

This application is based on Japanese Patent Applications Nos. 2006-040920 and 2006-040921, the contents of which are incorporated herein by reference.

### 2. DESCRIPTION OF RELATED ART

As a conventional implantation kit, a kit having the structure described in the online document "CELLPLEX™ TCP Synthetic Cancellous Bone", (2003, Wright Medical Technology Inc. (retrieved July 9, 2004), Internet URL http://www.wmt.com/Downloads/CellPlex_Brochure.pdf) has been known.

This known implantation kit is constructed by infiltrating a cylindrical container having removable caps at both ends with a scaffold material, such as tricalcium phosphate. Each cap at the end has either a bone-marrow injection hole for filling the cylindrical container with bone marrow or an air-release hole for releasing air when filling the cylindrical container with bone marrow.

To use this implantation kit, the tip of a syringe that is filled with bone marrow collected from a patient is connected to the bone-marrow injection hole, and the piston of the syringe is pressed. In this way, the bone marrow is injected from the syringe into the cylindrical container through the bone-marrow injection hole. At the same time, air escapes from the cylindrical container through the air-release hole formed in the other cap so that the injected bone marrow infiltrates the scaffold material. The bioprosthesis obtained by infiltrating the scaffold material with bone marrow is then pushed out using a piston that is attached after removing the caps.

However, the implantation kit having the structure described in the above "CELLPLEX^{™} TCP Synthetic Cancellous Bone" has a container with a relatively large diameter. Therefore, there is a problem in that, to implant the pushed out bioprosthesis into an affected site, a large incision must be made at the affected site. To solve this problem, the container must be constructed with a smaller diameter.

However, since the frictional force between the viscous bioprosthesis that is constructed by infiltrating the scaffold material with bone marrow and the inner wall of the container is great, a significant force must be applied to push out the bioprosthesis from the container when the diameter of the container is decreased. Therefore, when the diameter of the container is decreased, the length of the bioprosthesis accommodated in the container cannot be increased.

In this case, if the size of the container is matched with the volume of the bioprosthesis to be accommodated in the container, the container will be thin and short, making it difficult to handle the container. Thus, it is necessary to use a container that is long but accommodates a short bioprosthesis.

However, there is a problem in that, when pushing out the bioprosthesis from the container by inserting a piston that is disposed in an airtight manner against the inner wall of the container, only the bone marrow of the bioprosthesis is pushed out from the container by an air layer formed between the piston and the bioprosthesis.

As described above, when the container is formed with a small diameter, the length of the bioprosthesis accommodated in the container cannot be increased. Therefore, there is a problem in that the amount of bioprosthesis that can be implanted at once is limited.

### BRIEF SUMMARY OF THE INVENTION

The present invention has been conceived in light of the problems described above. Accordingly, it is an object of the present invention to provide a bioprosthesis preparation and implantation kit that is capable of implanting a bioprosthesis constituted by infiltrating a scaffold material held inside a container with body fluid, such as bone marrow, into an affected side without separating it from the body fluid, reducing the burden placed on a patient, and improving the ease-of-use.

Another object of the present invention is to provide a bioprosthesis implantation device that is capable of implanting a sufficient amount of viscous bioprosthesis into an affected site with a thin container, reducing the burden placed on a patient, and improving the ease-of-use.

To achieve the above-described objects, the present invention provides the following solutions.

A first aspect of the present invention provides a bioprosthesis preparation and implantation kit that includes a porous scaffold material; a cylindrical container configured to accommodate the scaffold material, the container having openings at first and second ends; a cap removably attached to the first end of the container so as to seal the opening at the first end; and a blocking member removably attached to the second end of the container so as to seal the opening at the second end. A through-hole for injecting liquid, such as bone marrow, is formed in the cap. The blocking member is inserted into the container and is provided with an insertion section for limiting the accommodation space for the scaffold material to a part near the cap. At least one air-release hole penetrating in the radial direction is formed in the container close to the tip of the insertion section of the blocking member attached to the container.

With this aspect, the bioprosthesis can be prepared inside the container by blocking both ends of the cylindrical container with the cap and the blocking member and infiltrating the scaffold material that is held inside the container with liquid, such as bone marrow, that is injected from the through-hole in the cap. Then, after opening the second end by removing the blocking member, for example, a piston is inserted into the container instead of the blocking member. In this state, the cap on the first end is removed. Since the container is formed as a linear cylinder, by merely pushing the piston, the bioprosthesis held inside the container can be pushed out from the first end of the container and can be directly implanted into an affected site.

In such a case, an air layer is formed between the bioprosthesis inside the container and the piston when the piston is inserted into the container. However, since the air in the air layer escapes through the air-release hole formed in the container in such a manner that the hole penetrates in the radial direction, the pressure in the air layer is prevented from excessively increasing. In this way, the liquid of the bioprosthesis is prevented from being discharged separately from the scaffold material.

Since the accommodating space of the scaffold material inside the container is limited to a part near the cap by inserting the blocking member, the bioprosthesis can be pushed out of the container with a relatively small pressure even when the diameter of the container is small, and the ease-of-use is improved. Moreover, even with a thin container, implantation of the bioprosthesis is possible without making a large incision at the affected site, and the burden placed on the patient can thus be reduced.

As a liquid to be injected, in addition to body fluid, such as bone marrow, growth factors and pharmaceutical, such as nutrients, may be used.

A second aspect of the present invention provides a bioprosthesis preparation and implantation kit including a porous scaffold material; a cylindrical container configured to accommodate the scaffold material, the container having openings at first and second ends; a cap removably attached to the first end of the container so as to seal the opening at the first end; and a blocking member removably attached to the second end of the container so as to seal the opening at the second end. A through-hole for injecting liquid, such as bone marrow, is formed in the cap. The blocking member is inserted into the container and is provided with an insertion section for limiting the accommodation space for the scaffold material to a part near the cap. At least one air-release groove extending from near the tip of the insertion section of the blocking member attached to the container to the opening on the side of a base is provided on the inner surface of the container.

According to this aspect, by allowing the air-release groove to function in the same way as the above-described air-release, a bioprosthesis constituted by infiltrating a scaffold material held inside a container with body fluid, such as bone marrow, can be implanted into an affected side without separating it from the body fluid, the burden placed on a patient can be reduced, and the ease-of-use can be improved.

The first and second aspects are advantageous in that the bioprosthesis constituted by infiltrating a scaffold material held inside a container with body fluid, such as bone marrow, can be implanted into an affected side without separating it from the body fluid, such as bone marrow, can be implanted into an affected site, the burden placed on a patient can be reduced, and the ease-of-use can be improved.

A third aspect of the present invention provides a bioprosthesis implantation device including a cylindrical container configured to accommodate a viscous bioprosthesis, the container having openings at both ends; and a piston configured to push the bioprosthesis inside the container and discharge the bioprosthesis from the opening at the forward end, the piston being inserted into the opening at the rear end, wherein the inner diameter of an accommodating space for the bioprosthesis gradually increases toward the opening at the forward end in the longitudinal direction.

According to this aspect, when the viscous bioprosthesis held inside the container is pushed by the piston inserted into the opening at the rear end, the bioprosthesis moves toward the opening at the forward end. Since the inner diameter of the accommodating space inside the container gradually increases toward the opening at the forward end in the longitudinal direction, the bioprosthesis moves in the direction that causes the bioprosthesis to be further separated from the inner surface of the container. As a result, the frictional force between the bioprosthesis and the inner surface of the container decreases, enabling discharge of the bioprosthesis from the opening at the forward end without applying an excessive pressure to the piston.

Thus, it is possible to make the container thin, and the bioprosthesis can thus be implanted without making a large incision at the affected site. As a result, the burden placed on the patient can be reduced. Since the frictional force between the bioprosthesis and the inner surface of the container decreases even though the container is thin, the length of the accommodating space for the bioprosthesis can be increased. As a result, a sufficient amount of bioprosthesis can be implanted at once.

According to this aspect, the accommodating space may have a tapered inner surface that widens toward the opening at the forward end.

In this way, as the bioprosthesis moves in the axial direction, the bioprosthesis becomes further separated from the entire tapered inner surface, causing a significant decrease in the frictional force. As a result, the external force to be applied to the piston can be reduced, and the bioprosthesis can be easily pushed out.

According to this aspect, a large-diameter section widening toward the opening at the forward end may be provided in at least part of the accommodating space.

In this way, as the bioprosthesis moves in the axial direction, the bioprosthesis becomes further separated from the large-diameter section, causing a significant decrease in the frictional force. As a result, the external force to be applied to the piston can be reduced, and the bioprosthesis can be easily pushed out. At the large-diameter section, the inner diameter may increase in steps or may continuously and monotonically increase.

According to this aspect, it is preferable that a sealing member configured to seal the gap between the piston and the inner surface of the container be provided on the piston, and the inner diameter of the accommodating space be increased to an extent that maintains the seal by the sealing member.

In this way, the seal provided by the sealing member between the piston and the inner surface of the container is maintained even when the piston is disposed inside the accommodating space. Therefore, the bioprosthesis can be completely discharged from the opening at the forward end of the container.

The third aspect of the present invention is advantageous in that a sufficient amount of viscous bioprosthesis can be implanted into an affected site with a thin container, the burden placed on a patient can be reduced, and the ease-of-use can be improved.

### BRIEF DESCRIPTION OF THE SEVERAL VIEWS OF THE DRAWINGS

Fig. 1 is a longitudinal cross-sectional view illustrating a bioprosthesis preparation and implantation kit according to a first embodiment of the present invention.

Fig. 2 is a longitudinal cross-sectional view illustrating the preparation of a bioprosthesis using the bioprosthesis preparation and implantation kit illustrated in Fig. 1.

Fig. 3 is a longitudinal cross-sectional view illustrating the injection of liquid into a container of the bioprosthesis preparation and implantation kit illustrated in Fig. 1.

Fig. 4 is a longitudinal cross-sectional view illustrating the prepared bioprosthesis accommodated in the container of the bioprosthesis preparation and implantation kit illustrated in Fig. 1.

Fig. 5 is a longitudinal cross-sectional view illustrating the removal of a cap from the container of the bioprosthesis preparation and implantation kit illustrated in Fig. 1.

Fig. 6 is a longitudinal cross-sectional view illustrating the insertion of a piston, after the removal of the cap, into the container of the bioprosthesis preparation and implantation kit illustrated in Fig. 1.

Fig. 7 is a longitudinal cross-sectional view illustrating the piston inserted into the container of the bioprosthesis preparation and implantation kit illustrated in Fig. 1.

Fig. 8 is a longitudinal cross-sectional view illustrating the cap removed from the container of the bioprosthesis preparation and implantation kit illustrated in Fig. 7.

Fig. 9 is a longitudinal cross-sectional view illustrating the bioprosthesis pushed out from the container of the bioprosthesis preparation and implantation kit illustrated in Fig. 8.

Fig. 10 is a longitudinal cross-sectional view illustrating a modification of the bioprosthesis preparation and implantation kit illustrated in Fig 1.

Fig. 11 is a longitudinal cross-sectional view illustrating the insertion of a piston into a container of the bioprosthesis preparation and implantation kit illustrated in Fig. 10.

Fig. 12 is a longitudinal cross-sectional view illustrating a bioprosthesis implantation device according to a second embodiment of the present invention.

Fig. 13 is a longitudinal cross-sectional view illustrating a preparation and implantation kit for preparing a bioprosthesis inside an accommodating space of the bioprosthesis implantation device illustrated in Fig. 12.

Fig. 14 is a longitudinal cross-sectional view illustrating the preparation of a bioprosthesis using the preparation and implantation kit illustrated in Fig. 13.

Fig. 15 is a longitudinal cross-sectional view illustrating the injection of liquid into the container of the preparation and implantation kit illustrated in Fig. 13.

Fig. 16 is a longitudinal cross-sectional view illustrating the prepared bioprosthesis accommodated in the container of the preparation and implantation kit illustrated in Fig. 13.

Fig. 17 is a longitudinal cross-sectional view illustrating the removal of a cap from the container of the preparation and implantation kit illustrated in Fig. 13.

Fig. 18 is a longitudinal cross-sectional view illustrating the insertion of a piston, after the removal of the cap, into the container of the preparation and implantation kit illustrated in Fig. 13.

Fig. 19 is a longitudinal cross-sectional view illustrating the cap removed from the bioprosthesis implantation device illustrated in Fig. 12.

Fig. 20 is a longitudinal cross-sectional view illustrating the bioprosthesis being pushed out from the bioprosthesis implantation device illustrated in Fig. 12. for implantation.

Figs. 21A and 21B are schematic views illustrating the push-out operation of the bioprosthesis by the bioprosthesis implantation device illustrated in Fig. 12.

Figs. 22A and 22B are schematic views illustrating modifications of the bioprosthesis implantation device illustrated in Fig. 12.

### DETAILED DESCRIPTION OF THE INVENTION

First Embodiment

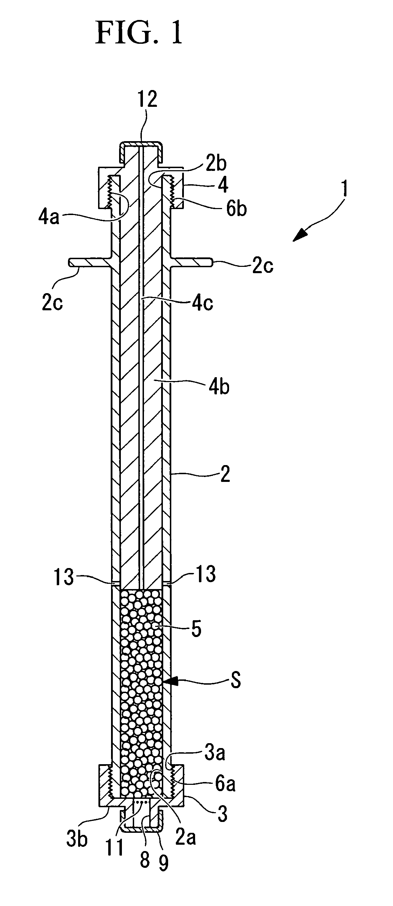

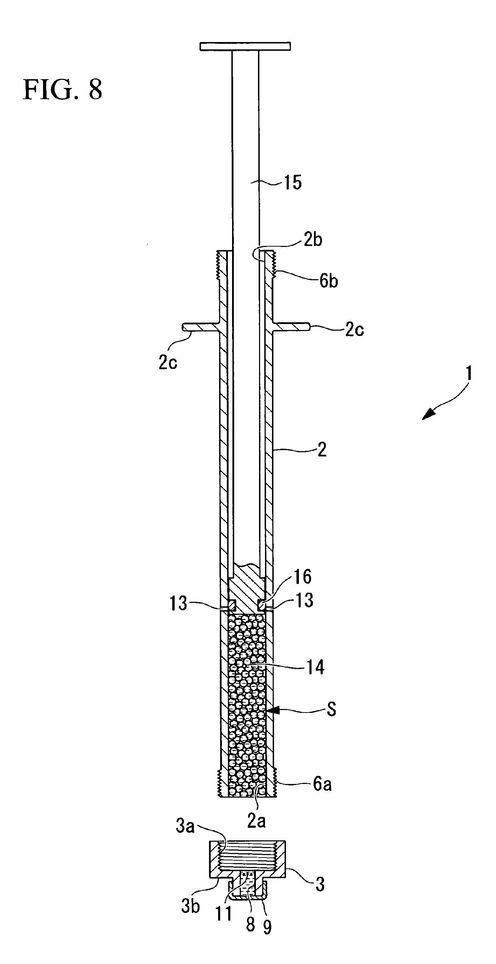

A bioprosthesis preparation and implantation kit 1 according to a first embodiment of the present invention will be described below with reference to Figs. 1 to 9.

As illustrated in Fig. 1, the bioprosthesis preparation and implantation kit 1 according to this embodiment includes a cylindrical container 2 having open ends and first and second caps (blocking members) 3 and 4 for blocking the ends of the container 2. The inside of the bioprosthesis preparation and implantation kit 1 is filled with a scaffold material 5. The container 2, for example, is made of a transparent material, such as glass or resin, so that the inside can be observed from the outside.

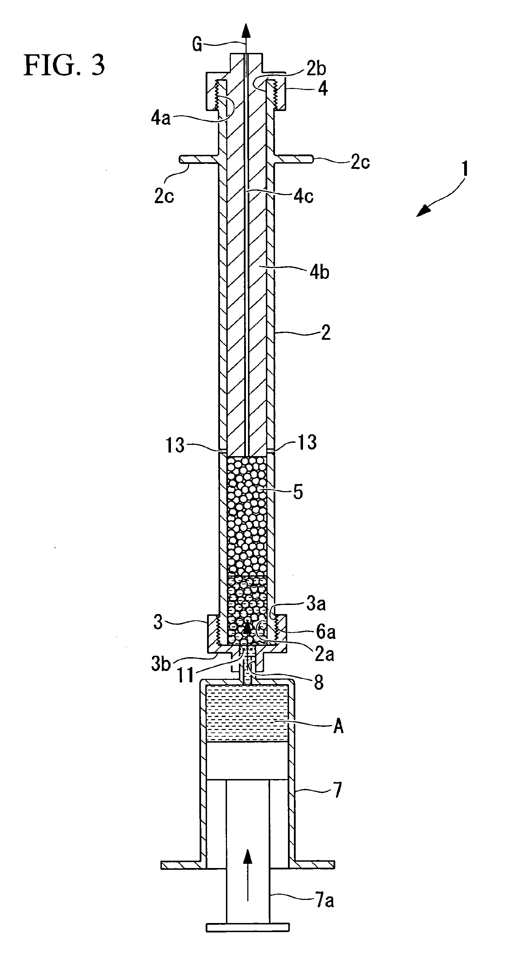

The first cap 3 is used to block an opening 2a at one of the ends of the container 2 and includes a female screw 3a that is engaged with a male screw 6a provided on the outer end surface of the container 2. The first cap 3 also has a through-hole 8 in which a syringe 7 (refer to Fig. 3) can be inserted at the center area of an end plate 3b disposed at a position that blocks the opening 2a of the container 2. The through-hole 8 is blocked by a removable plug 9.

The first cap 3 includes a mesh member 11 that prevents the scaffold material 5 in the container 2 from entering the through-hole 8 when the first cap 3 is attached to the container 2 and that allows liquid A, such as bone marrow injected by the syringe 7, to pass through.

The second cap 4 is used to block the opening 2b at the other end of the container 2 and includes a female screw 4a that is engaged with a male screw 6b provided on the outer end surface of the container 2. The second cap 4 includes an insertion section 4b that is engaged with the opening 2b of the container 2 over a predetermined depth.

By inserting the insertion section 4b of the second cap 4 into the container 2, the accommodating space S for the scaffold material 5 is defined near the first cap 3 over a limited length inside the container 2.

A through-hole 4c is formed in the center of the second cap 4, penetrating in the longitudinal direction. The through-hole 4c is blocked by a removable plug 12.

Air-release holes 13 that penetrate the sidewall of the container 2 in the radial directions are formed near the tip of the insertion section 4b of the second cap 4 that is inserted into the container 2. When the second cap 4 is fully engaged with the container 2, the air-release holes 13 are blocked by the insertion section 4b. In contrast, when the second cap 4 is loosened, the air-release holes 13 are immediately unblocked and air is introduced into the accommodating space S for the scaffold material 5. Grips 2c are provided on the container 2 for holding the container 2 with one's fingers.

The scaffold material 5 held inside the container 2 is, for example, granular porous β-tricalcium phosphate (β-TCP). The scaffold material 5 is disposed inside the container 2, whose openings 2a and 2b at both ends are blocked by the first and second caps 3 and 4, respectively, and fills the entire accommodating space S defined by the tip of the insertion section 4b and the first cap 3.

A method of preparing and implanting a bioprosthesis 14 by using the bioprosthesis preparation and implantation kit 1 according to this embodiment, having the above-described structure, will be described below.

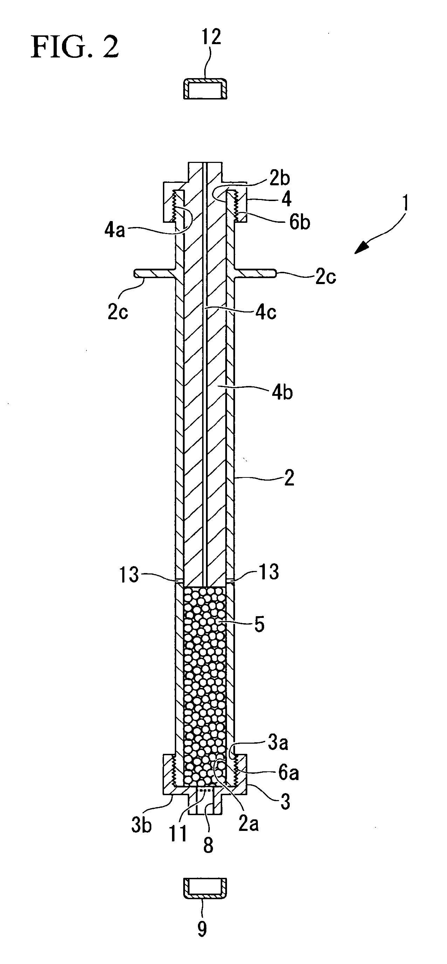

To prepare the bioprosthesis 14 using the bioprosthesis preparation and implantation kit 1 according to this embodiment, first, as shown in Fig. 2, the plugs 9 and 12 blocking the through-holes 8 and 4c of the first and second caps 3 and 4 are removed. Next, as shown in Fig. 3, the syringe 7 containing the liquid A collected from a patient, such as body fluid, e.g., bone marrow or peripheral blood, or pharmaceuticals, such as growth factors and nutrients, is inserted into the through-hole 8 of the first cap 3.

Since the mesh member 11 prevents the scaffold material 5 inside the container 2 from entering the space inside the through-hole 8, the tip of the syringe 7 can be inserted into the through-hole 8 without being blocked by the scaffold material 5. In this state, by pressing a piston 7a of the syringe 7, the liquid A inside the syringe 7 is supplied into the container 2.

At this time, as shown in Fig. 3, it is desirable to inject the liquid A upward from the through-hole 8 in the first cap 3 that is facing downward. In this way, the liquid A can be injected from the syringe 7 as it builds up inside the container 2. At the same time, air G inside the container 2 is released to the outside of the container 2 through the through-hole 4c of the second cap 4 that is provided in the upper section of the container 2. In this way, the liquid A can be supplied into the container 2 without any resistance and can infiltrate the scaffold material 5 inside the container 2.

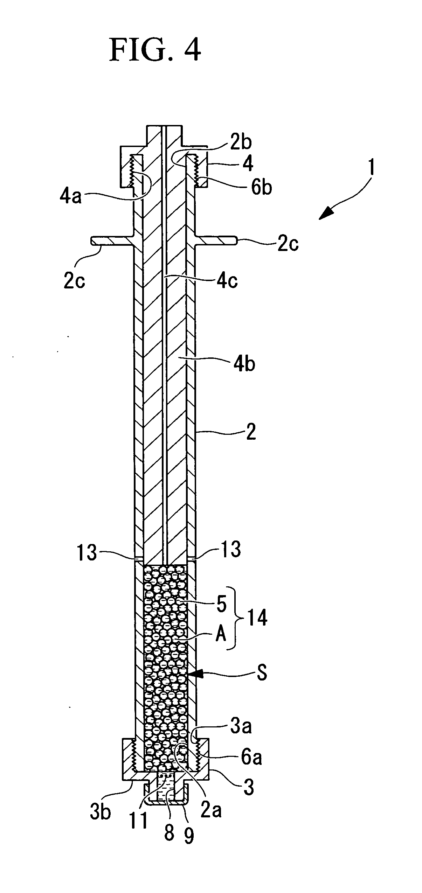

With the bioprosthesis preparation and implantation kit 1 according to this embodiment, the bioprosthesis 14 can be prepared so that the liquid A sufficiently infiltrates the scaffold material 5, as shown in Fig. 4.

When the liquid A that infiltrates the bioprosthesis 14 that is prepared using the bioprosthesis preparation and implantation kit 1 according to this embodiment consists of body fluid, such as bone marrow, including cells, after the bioprosthesis 14 is implanted to an affected site, the cells infiltrating the bioprosthesis 14 can also grow from the inside of the bioprosthesis 14 to quickly restore the affected site.

When the liquid A is a pharmaceutical solution, the solution can act on cells so that cell growth is enhanced also inside the bioprosthesis 14.

Next, a method of implanting the bioprosthesis 14, prepared as described above, to an affected site will be described below.

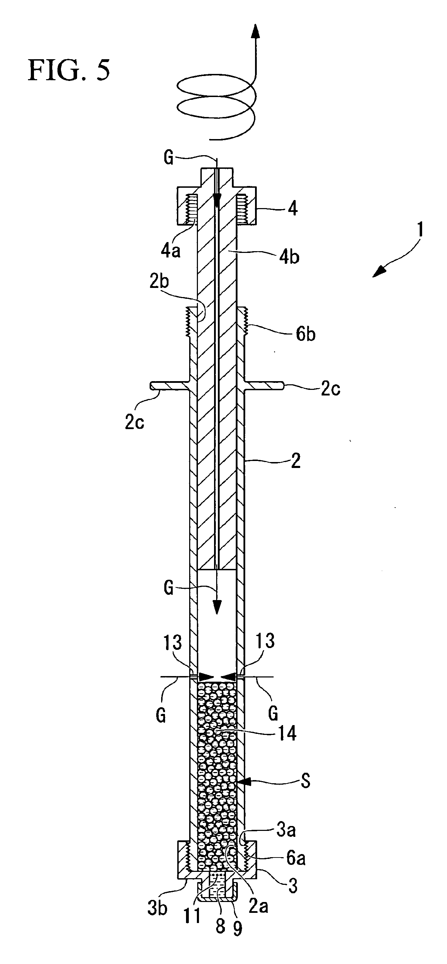

To implant the bioprosthesis 14 prepared using the bioprosthesis preparation and implantation kit 1 according to this embodiment to an affected site, first, as shown in Fig. 4, the through-hole 8 in the first cap 3 is blocked with the plug 9, and then, as shown in Fig. 5, the second cap 4 is removed from the container 2.

Since the second cap 4 is engaged with the container 2 with the screws 4a and 6a, the second cap 4 can be easily removed by turning the second cap 4 in the direction that loosens the screws 4a and 6a. At this time, air G is introduced into the container 2 through the through-hole 4c when the second cap 4 is moved, since the through-hole 4c is formed in the second cap 4. Since the air-release holes 13 are formed in the container 2, air G is introduced into the container 2 through the air-release holes 13 when the second cap 4 starts moving and the air-release holes 13 are released. Consequently, the second cap 4 can be easily removed without depressurizing the inside of the container 2 by the movement of the second cap 4.

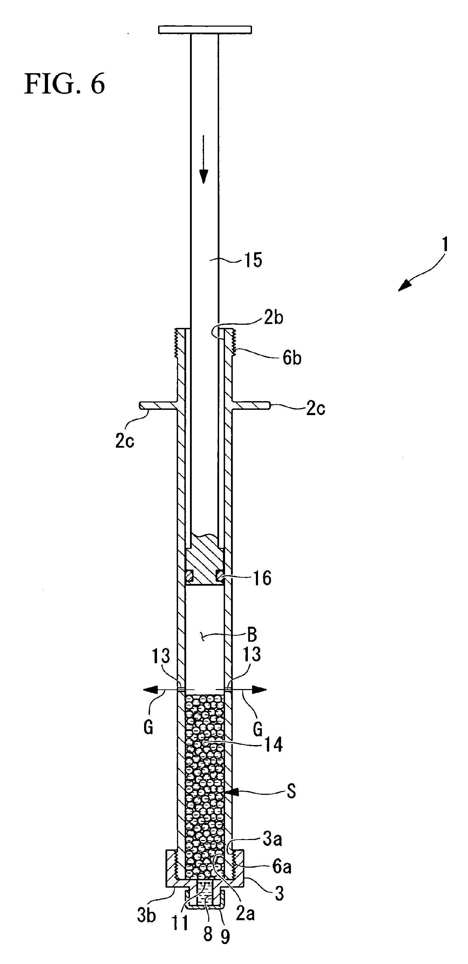

To replace the second cap 4 removed in such a manner as described above, a piston 15 is inserted into the opening 2b of the container 2, as illustrated in Fig. 6. The piston 15 is provided with a sealing member, such as an O-ring 16, at the tip thereof and is movable while maintaining air-tightness with the inner surface of the container 2.

Although, consequently, an air layer B is formed between the piston 15 and the bioprosthesis 14, since the air-release holes 13 are formed in the container 2 in the bioprosthesis preparation and implantation kit 1 according to this embodiment, the air G inside the container 2 is released to the outside through the air-release holes 13 when the piston 15 is pushed into the container 2. Accordingly, the piston 15 can be inserted into the container 2 without excessively increasing the pressure inside the container 2.

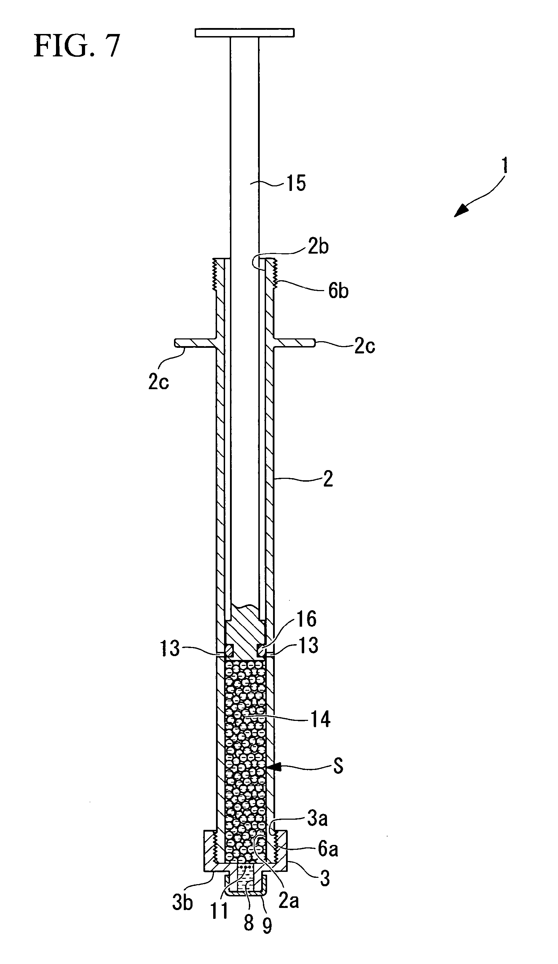

The piston 15 can be inserted into the container 2 until the air-release holes 13 of the container 2 are blocked by the O-ring 16 provided at the tip of the piston 15. Since the air-release holes 13 are formed near the end surface of the bioprosthesis 14, as shown in Fig. 7, the piston 15 can be inserted into the container 2 until the air layer B formed between the bioprosthesis 14 and the piston 15 substantially disappears.

As a result, the liquid A, such as bone marrow, included in the bioprosthesis 14 can be prevented from first being released from the opening 2a of the container 2 when the piston 15 is inserted into the container 2 due to a pressure rise in the air layer B inside of the container 2. Furthermore, the mixture of the scaffold material 5 and the liquid A constituting the bioprosthesis 14 can be discharged together.

Then, in this state, the first cap 3 is removed from the container 2. Since the first cap 3 is engaged with the container 2 with the screws 3a and 6a, the first cap 3 can be easily removed by turning the first cap 3 in the direction that loosens the screws 3a and 6a.

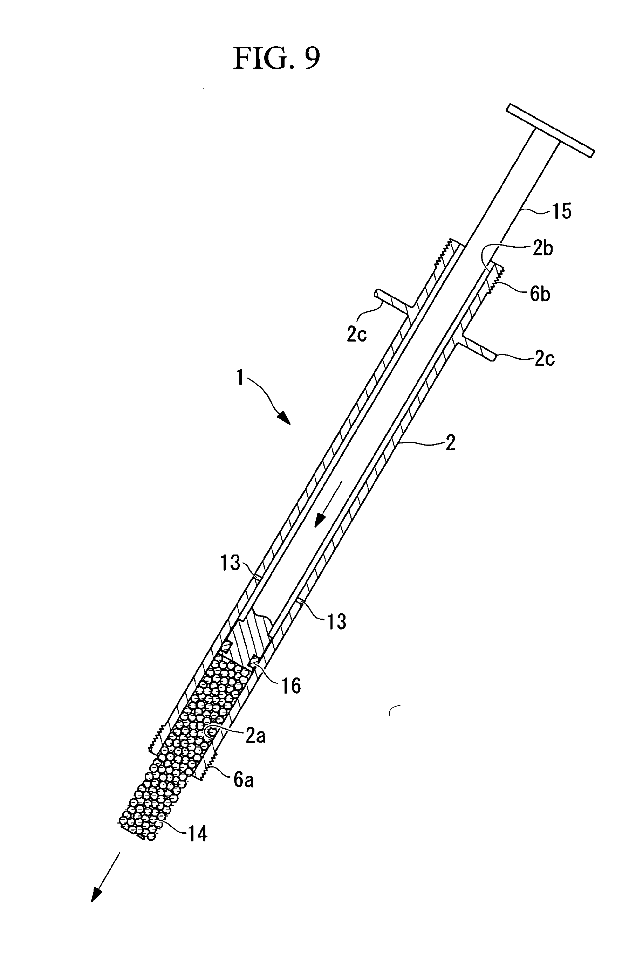

Then, as shown in Fig. 9, by moving the tip of the container 2 close to the affected site and pushing down the piston 15, the bioprosthesis 14 inside the container 2 is pushed out from the tip of the container 2 and is implanted to the affected site.

In this case, the bioprosthesis 14 with a short length can be prepared with the bioprosthesis preparation and implantation kit 1 according to this embodiment since the accommodating space S for the bioprosthesis 14 is limited to only a section of the container 2 by inserting the insertion section 4b of the second cap 4 into the container 2. Thus, even when the diameter of the container 2 is reduced, the external force applied to the piston 15 so as to push out the bioprosthesis 14 is not excessively increased, and the ease of implantation is improved. Moreover, there is an advantage in that, even when the container 2 is thin and the bioprosthesis 14 is short, the ease-of-use for the injection process of the liquid A, such as bone marrow, and the implantation process of the bioprosthesis 14 can be increased.

When the granular scaffold material 5 is directly implanted to an affected site, there is a problem in that the granular scaffold material 5 attaches to areas around the affected site. However, according to this embodiment, the scaffold material 5 is held together by the liquid A. As a result, the ease-of-implantation of the bioprosthesis 14 is improved, and the scaffold material 5 can be prevented from being attached to areas around the affected site.

By changing the amount by which the piston 15 is pushed, the amount of bioprosthesis 14 to be implanted can be adjusted depending on the size of the affected site.

In this embodiment, granular porous β-TCP is used as the scaffold material 5. Instead of this, however, other porous biocompatible materials may be used as the scaffold material 5. Furthermore, the number of air-release holes 13 penetrating the wall of the container 2 may be one or more.

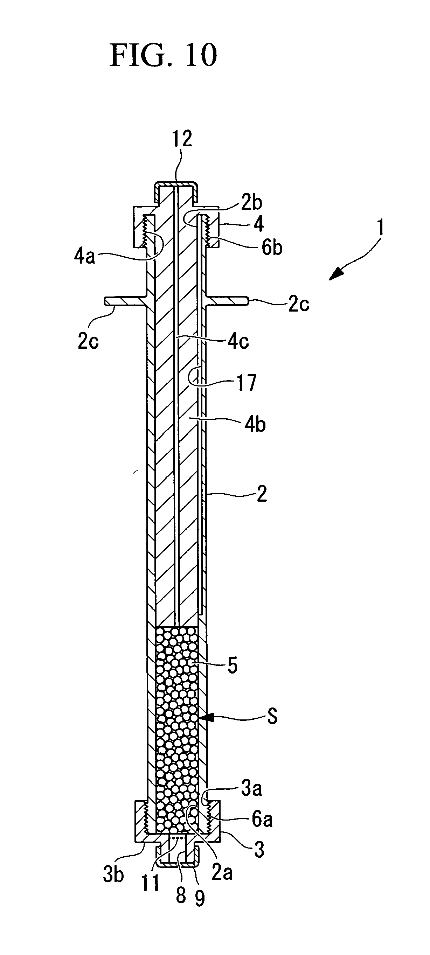

In this embodiment, the air-release holes 13 penetrating the wall of the container 2 are provided. Instead of this, however, an air-release groove 17 may be formed on the inner surface of the container 2, as shown in Fig. 10.

The air-release groove 17 extends continuously from near the tip of the insertion section 4b of the second cap 4 that is attached to the container 2 to the opening 2b that is blocked by the second cap 4. When the second cap 4 is fully engaged with the container 2, the air-release groove 17 is blocked by the insertion section 4b of the second cap 4. However, when the second cap 4 is loosened, the air-release groove 17 is immediately connected to the accommodating space S inside the container 2.

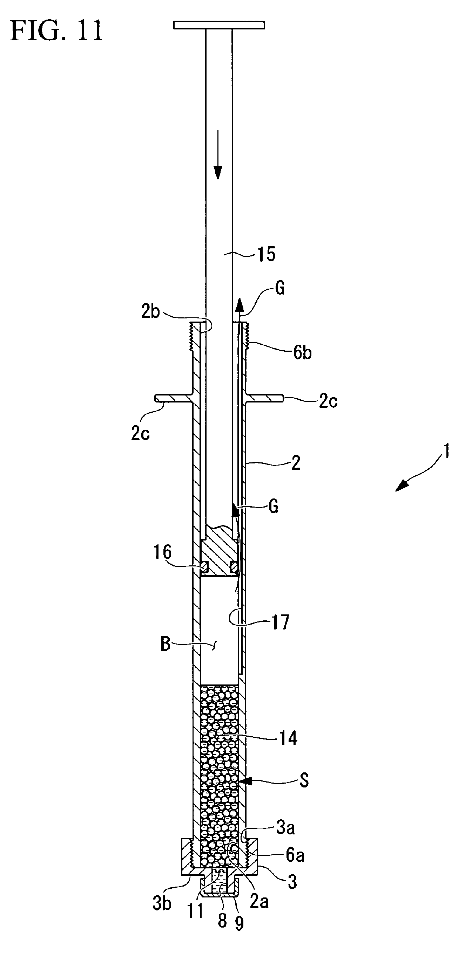

Accordingly, when the piston 15 is inserted into the container 2 to replace the second cap 4, the air layer B formed between the piston 15 and the bioprosthesis 14 is released to the outside space through the air-release groove 17, as illustrated in Fig. 11. Therefore, even when the piston 15 is pushed into the container 2, the pressure of the air layer B inside the container 2 does not excessively increase. Thus, in the same manner as described above, the bioprosthesis 14 and the liquid A can be pushed out of the container 2 without being separated.

### Second Embodiment

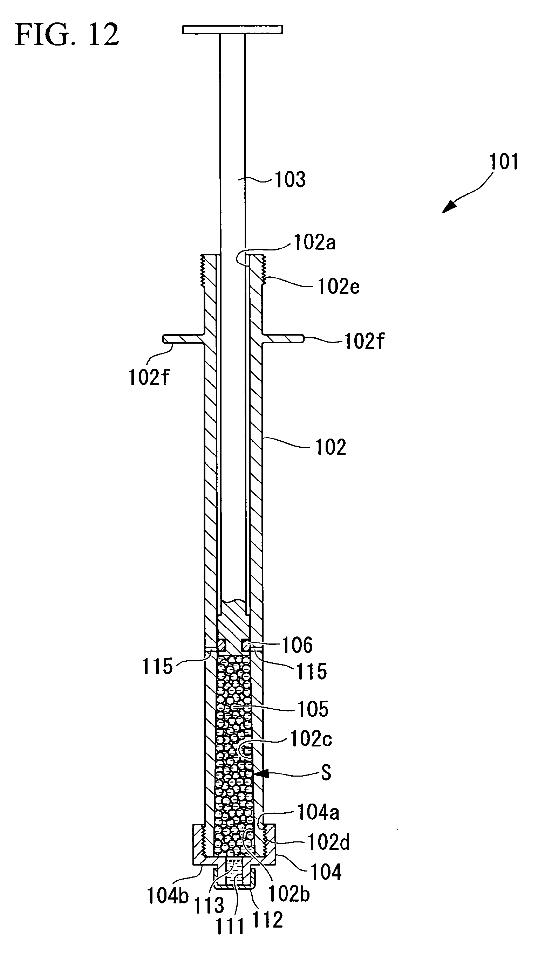

A bioprosthesis implantation device 101 according to a second embodiment of the present invention will be described below with reference to Figs. 12 to 22.

As illustrated in Fig. 12, the bioprosthesis implantation device 101 according to this embodiment includes a substantially cylindrical container 102 having open ends and a piston 103 that is inserted into a rear opening 102a of the cylindrical container 102. As shown in Fig. 12, a forward opening 102b is blocked by a first cap 104.

The container 102 includes an accommodating space S that is provided for accommodating a bioprosthesis 105 on the side of the forward opening 102b. The accommodating space S has a tapered inner surface 102c having an inner diameter that gradually increasing in the axial direction from the rear opening 102a to the forward opening 102b. The container 102 is provided with grips 102f for holding the container 102 with one's fingers.

A sealing member, such as an O-ring 106, is provided at the tip of the piston 103. The O-ring 106 seals the gap between the piston 103 and the inner surface of the container 102. In this embodiment, the O-ring 106 may be disposed at other positions in the axial direction of the accommodating space S to seal the gap between the piston 103 and the inner surface of the container 102.

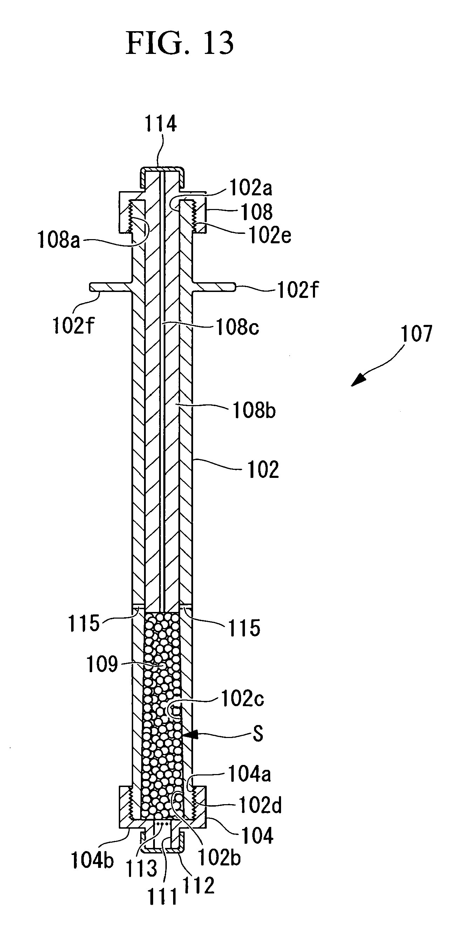

The bioprosthesis implantation device 101 is provided as, for example, a preparation implantation kit 107 and the piston 103 that is provided separately from the preparation implantation kit 107, as illustrated in Fig 13. The preparation implantation kit 107 includes the first cap 104 and a second cap 108 for blocking both ends of the container 102. The accommodating space S inside the container 102 is filled with a scaffold material 109. The container 102, for example, is made of a transparent material, such as glass or resin, so that the condition of the inside can be observed from the outside.

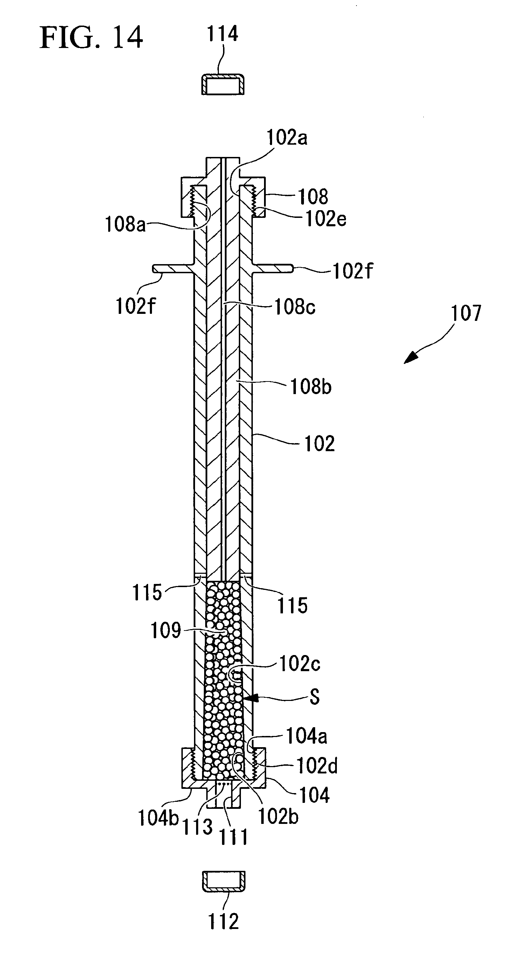

The first cap 104 is used to block the forward opening 102b of the container 102 and includes a female screw 104a that is engaged with a male screw 102d provided on the outer end surface of the container 102. The first cap 104 also has a through-hole 111 in which a syringe 110 (refer to Fig. 14) can be inserted at the center area of an end plate 104b disposed at a position that blocks the forward opening 102b of the container 102. The through-hole 111 is blocked by a removable plug 112.

The first cap 104 includes a mesh member 113 that prevents the scaffold material 109 in the container 102 from entering the through-hole 111 when the first cap 104 is attached to the container 102 and that transmits liquid A, such as bone marrow, injected by the syringe 110.

The second cap 108 is used to block the rear opening 102a of the container 102 and includes a female screw 108a that is engaged with a male screw 102e provided on the outer end surface of the container 102. The second cap 108 includes an insertion section 108b that is engaged with the rear opening 102a of the container 102 over a predetermined depth.

By inserting the insertion section 108b of the second cap 108 into the container 102, the accommodating space S for the scaffold material 109 is defined near the first cap 104 over a limited length inside the container 102.

A through-hole 108c is formed in the center of the second cap 108, penetrating in the longitudinal direction. The through-hole 108c is blocked by a removable plug 114.

Air-release holes 115 that penetrate the sidewall of the container 102 in the radial directions are formed near the tip of the insertion section 108b of the second cap 108 that is inserted into the container 102. When the second cap 108 is fully engaged with the container 102, the air-release holes 115 are blocked by the insertion section 108b. In contrast, when the second cap 108 is loosened, the air-release holes 115 are immediately unblocked and air is introduced into the accommodating space S for the scaffold material 109.

The scaffold material 109 held inside the container 102 is, for example, granular porous β-tricalcium phosphate (β-TCP). The scaffold material 109 is disposed inside the container 102, whose openings 102a and 102b at both ends are blocked by the first and second caps 104 and 108, respectively, and fills the entire accommodating space S defined by the tip of the insertion section 108b and the first cap 104.

A method of preparing and implanting the bioprosthesis 105 by using the preparation implantation kit 107 according to this embodiment, having the above-described structure, will be described below.

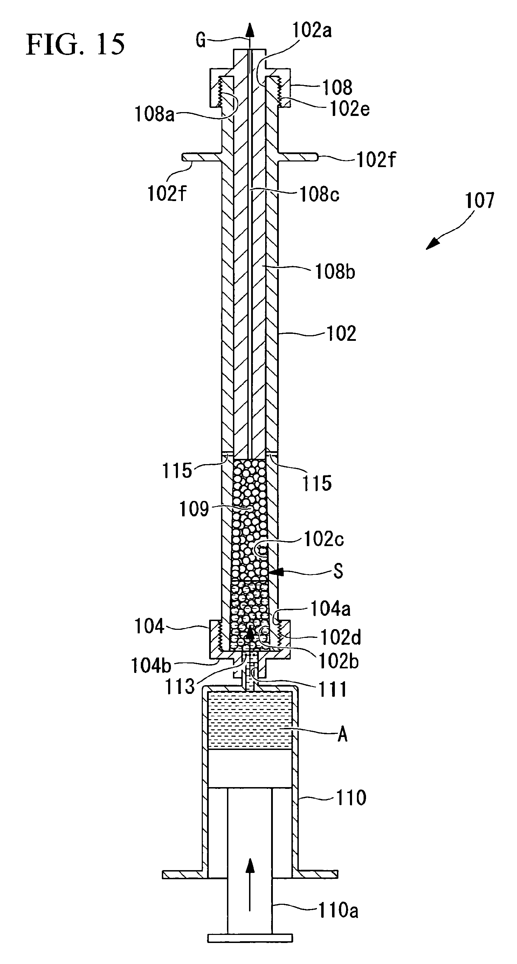

To prepare the bioprosthesis 105 using the preparation implantation kit 107 according to this embodiment, first, as shown in Fig. 14, the plugs 112 and 114 blocking the through-holes 111 and 108c of the first and second caps 104 and 108 are removed. Next, as shown in Fig. 15, the syringe 110 containing the liquid A collected from a patient, such as body fluid, e.g., bone marrow or peripheral blood, or pharmaceuticals, such as growth factors and nutrients, is inserted into the through-hole 111 of the first cap 104.

Since the mesh member 113 prevents the scaffold material 109 inside the container 102 from entering the space inside the through-hole 111, the tip of the syringe 110 can be inserted into the through-hole 111 without being blocked by the scaffold material 109. In this state, by pressing a piston 110a of the syringe 110, the liquid A inside the syringe 110 is supplied into the container 102.

At this time, as shown in Fig. 15, it is desirable to inject the liquid A upward from the through-hole 111 in the first cap 104 that is facing downward. In this way, the liquid A can be injected from the syringe 110 as it builds up inside the container 102. At the same time, air G inside the container 102 is released to the outside of the container 102 through the through-hole 108c of the second cap 108 that is provided in the upper section of the container 102. In this way, the liquid A can be supplied into the container 102 without any resistance and can infiltrate the scaffold material 109 inside the container 102.

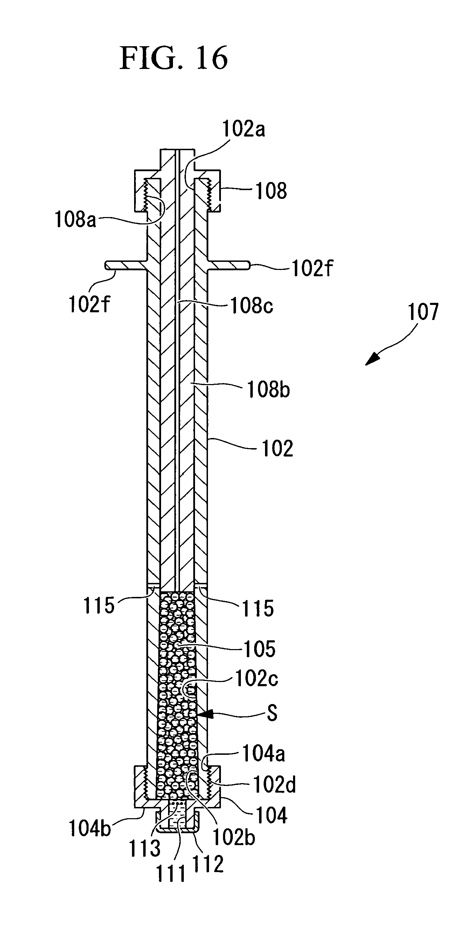

With the preparation implantation kit 107 according to this embodiment, the bioprosthesis 105 can be prepared so that the liquid A sufficiently infiltrates the scaffold material 109, as shown in Fig. 16.

When the liquid A that infiltrates the bioprosthesis 105 prepared using the preparation implantation kit 107 consists of body fluid, such as bone marrow, including cells, after the bioprosthesis 105 is implanted to an affected site, the cells infiltrating the bioprosthesis 105 can also grow from the inside of the bioprosthesis 105 to quickly restore the affected site.

When the liquid A is a pharmaceutical solution, the solution can act on cells so that cell growth is enhanced also inside the bioprosthesis 105.

Next, a method of implanting the bioprosthesis 105, prepared as described above, to an affected site will be described below.

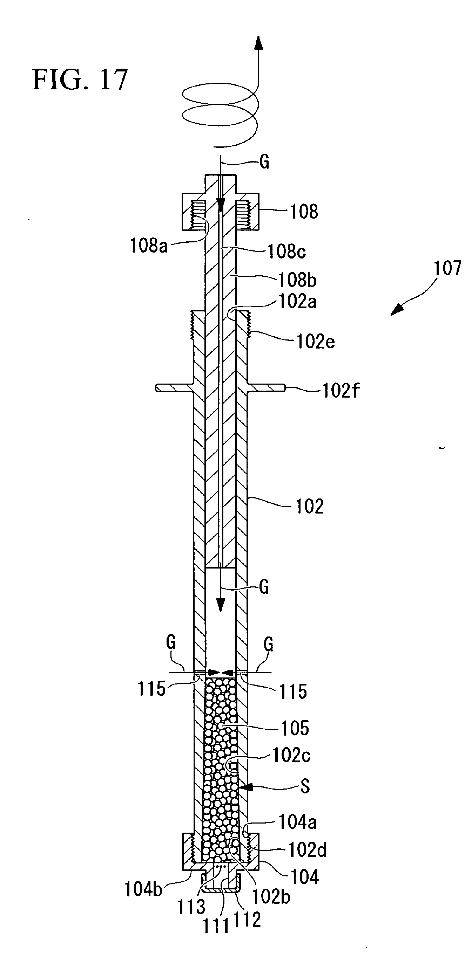

To implant the bioprosthesis 105 prepared using the preparation implantation kit 107 according to this embodiment to an affected site, first, as shown in Fig. 16, the through-hole 111 in the first cap 104 is blocked with the plug 112, and then, as shown in Fig. 17, the second cap 108 is removed from the container 102.

Since the second cap 108 is engaged with the container 102 with the screws 108a and 102e, the second cap 108 can be easily removed by turning the second cap 108 in the direction that loosens the screws 108a and 102e. At this time, air G is introduced into the container 102 through the through-hole 108c when the second cap 108 is moved, since the through-hole 108c is formed in the second cap 108. Since the air-release holes 115 are formed in the container 102, air G is introduced into the container 102 through the air-release holes 115 when the second cap 108 starts moving and the air-release holes 115 are released. Consequently, the second cap 108 can be easily removed without depressurizing the inside of the container 102 by the movement of the second cap 108.

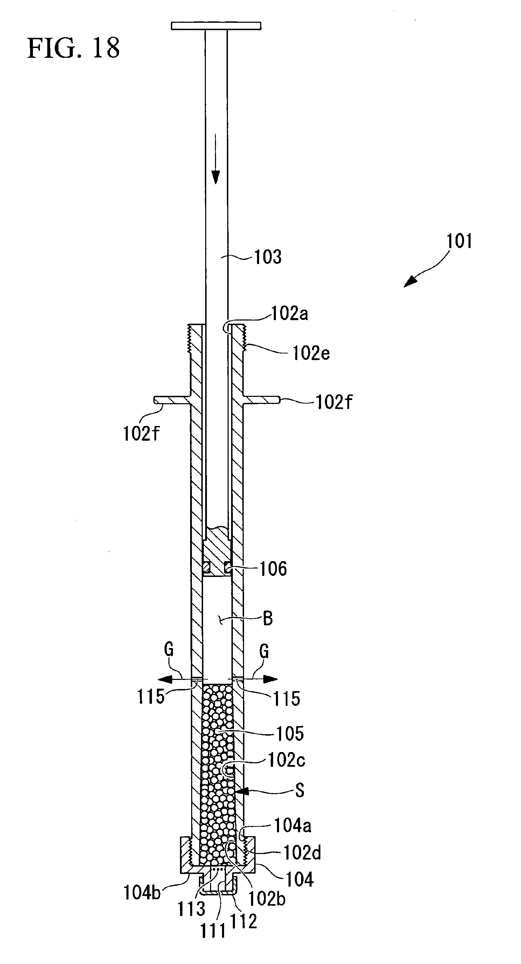

To replace the second cap 108 removed in such a manner as described above, the piston 103 is inserted into the rear opening 102a of the container 102, as illustrated in Fig. 18.

Although, consequently, an air layer B is formed between the piston 103 and the bioprosthesis 105, since the air-release holes 115 are formed in the container 102 in the bioprosthesis implantation device 101 according to this embodiment, the air G inside the container 102 is released to the outside through the air-release holes 115 when the piston 103 is pushed into the container 102. Accordingly, the piston 103 can be inserted into the container 102 without excessively increasing the pressure inside the container 102.

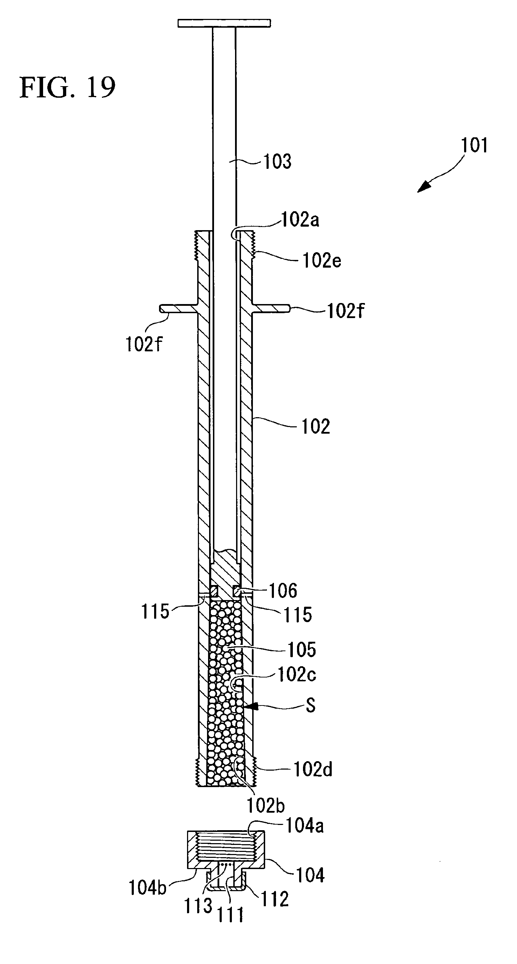

The piston 103 can be inserted into the container 102 until the air-release holes 115 of the container 102 are blocked by the O-ring 106 provided at the tip of the piston 103. Since the air-release holes 115 are formed near the end surface of the bioprosthesis 105, as shown in Fig. 19, the piston 103 can be inserted into the container 102 until the air layer B formed between the bioprosthesis 105 and the piston 103 substantially disappears.

As a result, the liquid A, such as bone marrow, included in the bioprosthesis 105 can be prevented from being released first from the forward opening 102b of the container 102 when the piston 103 is inserted into the container 102 due to a pressure rise in the air layer B inside of the container 102. Furthermore, the mixture of the scaffold material 109 and the liquid A constituting the bioprosthesis 105 can be discharged together.

Then, in this state, the first cap 104 is removed from the container 102. Since the first cap 104 is engaged with the container 102 with the screws 104a and 102b, the first cap 104 can be easily removed by turning the first cap 104 in the direction that loosens the screws 104a and 102b.

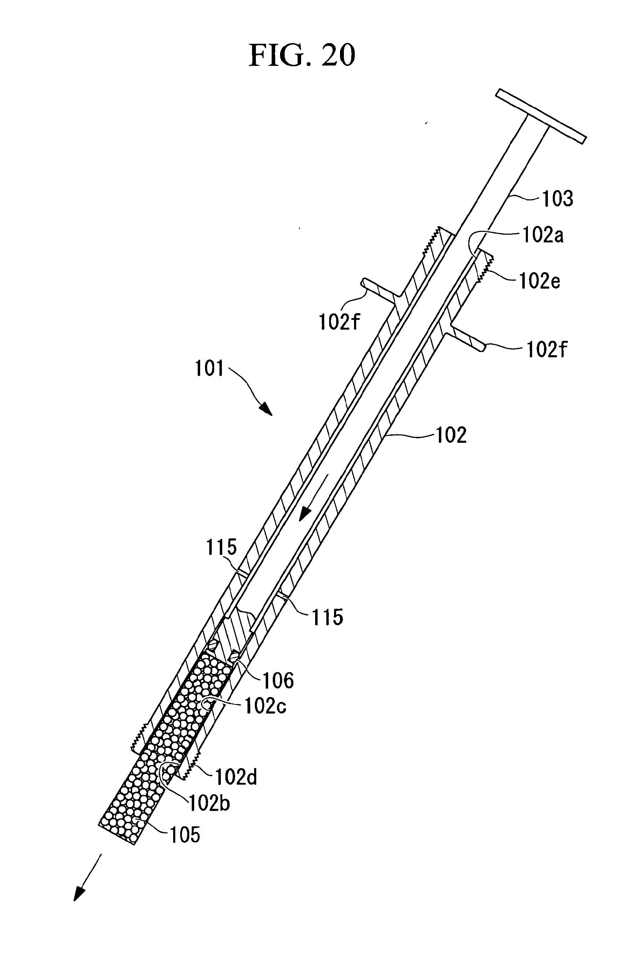

Then, as shown in Fig. 20, by moving the tip of the container 102 close to the affected site and pushing down the piston 103, the bioprosthesis 105 inside the container 102 is pushed out from the forward opening 102b of the container 102 and is implanted to the affected site.

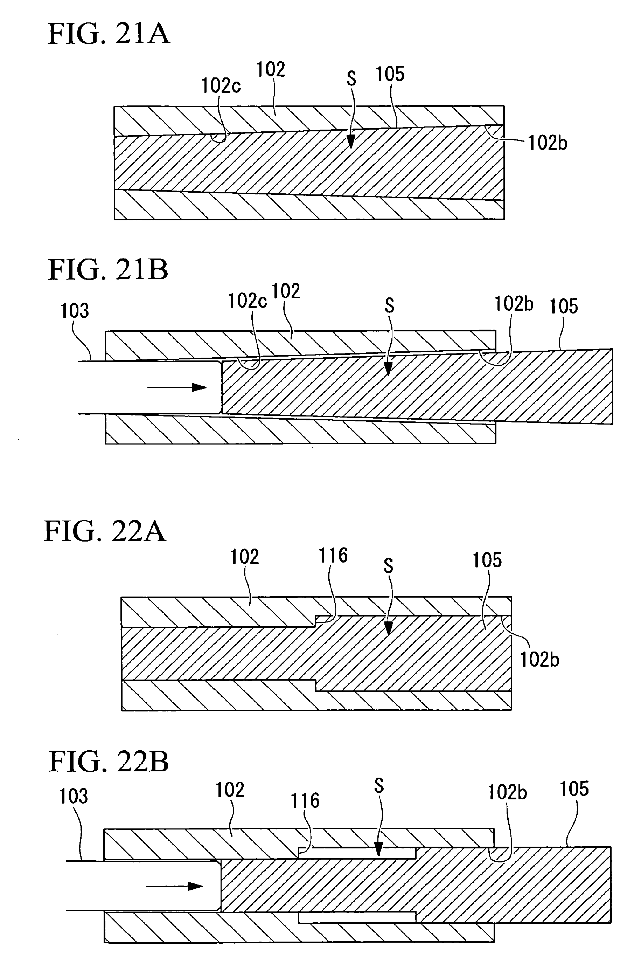

In this case, the bioprosthesis 105 inside the accommodating space S of the container 102 is in close contact with the inner surface 102c of the container 102 before being pushed out by the piston 103, as shown in Fig. 21A. With the bioprosthesis implantation device 101 according to this embodiment, since the accommodating space S for the bioprosthesis 105 has the tapered inner surface 102c having an inner diameter that gradually increase toward the forward opening 102b, the bioprosthesis 105 can be separated from the tapered inner surface 102c by slightly moving as a result of being pushed by the piston 103, as shown in Fig. 21B.

More specifically, although the viscous bioprosthesis 105 that is held inside the accommodating space S is in close contact with the tapered inner surface 102c before being pushed out by the piston 103, one it moves slightly, the frictional force between the piston 103 and the tapered inner surface 102c suddenly decreases, enabling the bioprosthesis 105 to be discharged from the forward opening 102b without applying a large pressure using the piston 103.

As a result, with the bioprosthesis implantation device 101 according to this embodiment, the container 102 may be configured with a small diameter. More specifically, with the bioprosthesis implantation device 101 according to this embodiment, even if pipe friction increases by reducing the diameter of the container 102, the pipe friction significantly decreases as soon as the bioprosthesis 105 starts moving. Therefore, excessive pressure is not required, and the bioprosthesis 105 can be easily implanted.

By reducing the diameter of the container 102, the bioprosthesis 105 can be implanted by inserting the container 102 into the affected site without making a large incision. Accordingly, the burden placed on the patient can be reduced. If the amount of the bioprosthesis 105 held inside the container 102 is reduced due to the small diameter of the container 102, the length of the accommodating space S can be extended to allow a sufficient amount of bioprosthesis 105 to be held inside the container 102.

More specifically, although pipe friction increases when the length of the accommodating space S is increased, as described above, with the bioprosthesis implantation device 101 according to this embodiment, the pipe friction is significantly reduced when the piston 103 is only pushed a little. Thus, even if the length of the bioprosthesis 105 is increased, the bioprosthesis 105 can be discharged with a relatively small pressure. This is advantageous in that the implantation process is facilitated.

According to this embodiment, since the O-ring 106 provided on the piston 103 maintains the air tightness between the piston 103 and the tapered inner surface 102c at any position in the accommodating space S, the bioprosthesis 105 can be discharged from the accommodating space S without leaking.

When the granular scaffold material 109 is directly implanted to an affected site, there is a problem in that the granular scaffold material 109 attaches to areas around the affected site. However, according to this embodiment, the scaffold material 109 is held together by the liquid A. As a result, the ease-of-implantation of the bioprosthesis 105 is improved, and the scaffold material 109 can be prevented from being attached to areas around the affected site.

By changing the amount by which the piston 103 is pushed, the amount of bioprosthesis 105 to be implanted can be adjusted depending on the size of the affected site.

In this embodiment, granular porous β-TCP is used as the scaffold material 109. Instead of this, however, other porous biocompatible materials may be used as the scaffold material 109. Furthermore, the number of the air-release holes 115 penetrating the wall of the container 102 may be one or more.

In this embodiment, the air-release holes 115 penetrating the wall of the container 102 are provided. Instead of this, however, an air-release groove may be formed on the inner surface of the container 102. The air-release groove extends continuously from near the tip of the insertion section 108b of the second cap 108 that is attached to the container 102 to the rear opening 102a that is blocked by the second cap 108. When the second cap 108 is fully engaged with the container 102, the air-release groove is blocked by the insertion section 108b of the second cap 108. However, when the second cap 108 is loosened, the air-release groove is immediately connected to the accommodating space S inside the container 102. In this way, similar to the air-release holes 115, the bioprosthesis 105 and the liquid A can be pushed out of the container 102 without being separated.

According to this embodiment, the tapered inner surface 102c is provided in the entire accommodating space S for the bioprosthesis 105. Instead of this, however, the tapered inner surface 102c may be provided only in certain regions of the accommodating space S. As described above, the tapered inner surface 102c is constructed by increasing the inner diameter of the accommodating space S at a constant rate. However, the embodiment is not limited thereto, and the inner surface of the accommodating space S may be shaped so that the inner diameter monotonically increases continuously or discontinuously at different rates.

Instead of the accommodating space S having the tapered inner surface 102c, as shown in Figs. 22A and 22B, a large-diameter section 116 where the diameter of the accommodating space S increases near the forward opening 102b may be provided. In this case, the contact area of the inner surface of the container 102 and the bioprosthesis 105 is very small in the state illustrated in Fig. 22B in which the bioprosthesis 105 has slightly moved by being pushed by the container 102 compared with the state illustrated in Fig. 22A in which the bioprosthesis 105 is held inside the accommodating space S. Therefore, frictional force is reduced, and only a small pressure needs to be applied to the piston 103.

Although the embodiments of the present invention have been described above, various modifications of the above-described embodiments may be provided within the scope of the present invention. Embodiments configured by partially combining the above-described embodiments are also included within the scope of the present invention.

## Claims

1. A bioprosthesis preparation and implantation kit comprising:
a porous scaffold material;
a cylindrical container configured to accommodate the scaffold material, the container having openings at first and second ends;
a cap removably attached to the first end of the container so as to seal the opening at the first end; and
a blocking member removably attached to the second end of the container so as to seal the opening at the second end,
wherein,
a through-hole for injecting liquid is formed in the cap,
the blocking member is inserted into the container and is provided with an insertion section for limiting the accommodation space for the scaffold material to a part near the cap, and
at least one air-release hole penetrating in the radial direction is formed in the container close to the tip of the insertion section of the blocking member attached to the container.

2. A bioprosthesis preparation and implantation kit comprising:
a porous scaffold material;
a cylindrical container configured to accommodate the scaffold material, the container having openings at first and second ends;
a cap removably attached to the first end of the container so as to seal the opening at the first end; and
a blocking member removably attached to the second end of the container so as to seal the opening at the second end,
wherein,
a through-hole for injecting liquid, is formed in the cap,
the blocking member is inserted into the container and is provided with an insertion section for limiting the accommodation space for the scaffold material to a part near the cap, and
at least one air-release groove extending from near the tip of the insertion section of the blocking member attached to the container to the opening on the side of a base is provided on the inner surface of the container.

3. A bioprosthesis implantation device comprising:
a cylindrical container configured to accommodate a viscous bioprosthesis, the container having openings at both ends; and
a piston configured to push the bioprosthesis inside the container and discharge the bioprosthesis from the opening at the forward end, the piston being inserted into the opening at the rear end,
wherein the inner diameter of an accommodating space for the bioprosthesis gradually increases toward the opening at the forward end in the longitudinal direction.

4. The bioprosthesis implantation device according to Claim 3, wherein the accommodating space has a tapered inner surface that widens toward the opening at the forward end.

5. The bioprosthesis implantation device according to Claim 3, wherein a large-diameter section widening toward the opening at the forward end is provided in at least part of the accommodating space.

6. The bioprosthesis implantation device according to Claim 3,
wherein a sealing member configured to seal a gap between the piston and the inner surface of the container is provided on the piston, and
wherein the inner diameter of the accommodating space is increased to an extent that maintains the seal by the sealing member.
